# EUROPEAN PATENT SPECIFICATION

(11) **EP 0 624 518 B1**
(45) Date of publication and mention of the grant of the patent: **24.09.1997**
(21) Application number: 94302392.9
(22) Date of filing: 05.04.1994
(51) Int. Cl.: B65B 55/10

(54) **Continuous sanitisation**
Kontinuierliche Sterilisation
Stérilisation continue

(30) Priority: 06.04.1993 GB 9307136
(43) Date of publication of application: 17.11.1994
(73) Proprietor: TOTAL PROCESS CONTAINMENT LIMITED, Elstead, Surrey GU8 6LB (GB)
(72) Inventor: McDonald, Austin Patrick, c/o Total Process, Elstead, Surrey GU8 6LB (GB); Wilkins, Julian James, Near Fakenham, Norfolk NR21 0HQ (GB)
(74) Representative: Silverman, Warren

(56) References cited:
- EP-A- 0 013 132
- CH-A- 530 307
- DE-A- 3 303 128
- US-A- 3 086 336
- US-A- 4 707 334

## Description

This invention relates to continuous sanitisation system and more particularly to the sanitisation of packs themselves containing sterile containers for sterile injectable material so as to prevent cross-contamination by the packs of the containers during handling of the packs to remove the containers therefrom.

In the medical products industry, pre-packed barrels containing vaccine are produced which are to be utilised in syringes for injection of the vaccine. Prior to filling, the barrels are frequently transported in packs holding, say, 100 such barrels arranged in an array 10 x 10 such that the barrels do not come into contact during transport. The packs for transporting the barrels from the barrel manufacturers typically consist of a plastics container faced with a Tyvek (Registered Trade Mark) panel which can easily be pulled off to provide access to the barrels for their removal from the pack prior to filling. "Tyvek" is a suitable material for a removable cover, insofar as preliminary ethylene oxide sterilisation of the entire pack of barrels is concerned. Large numbers of such packs may need to be produced in a sterile condition for despatch for filling by vaccine producers within a short time. Influenza vaccines are cultured over a short period of time once the required mixture of strains has been identified and the product then has to be on the market by a particular target date in the autumn. This has led to a need for a rapid, reliable and environmentally safe means of effecting sanitisation of the packs. In this connection, sanitisation means reduction in micro concentration to no more than 10³ microbes/m³. In many circumstances, however, a higher degree of microbe kill is desirable or even required, this being termed sterilisation and corresponds to a reduction in microbe concentration to below 10⁶ microbes/m³.

Sanitisation technology, and sterilization technology in which higher standards are set, has developed, at least insofar as continuous throughput of containers is required primarily in the food processing and packaging industry. Examples of developments in this field appear in GB-A-669,312. The use of isolating chambers in sterilization processes is known, *inter alia,* from US-A-4 707 334 wherein it is proposed that a barrier be established about a conveyor which transports articles to be sterilized so that the articles may be conveyed from the ambient atmosphere into a chamber where the articles are subject to action of a toxic sterilizing agent. The apparatus also isolates the sterilizing chamber from the ambient atmosphere by means of airlocks and the provision therein of plural zones of different pressures. These prevent toxic material from reaching the ambient atmosphere by having incoming gas flows being directed opposed to flows from the sterilization chamber. This procedure however detracts from the overall efficiency of the sterilization procedure which is to be carried out as amounts of spent sterilization medium are allowed to accumulate rather than be replenished.

It is an object of this invention to provide a sanitisation method in which the concentration of sterilization medium in the sanitisation chamber is enhanced.

According to the present invention there is provided a continuous sanitisation method which comprises supplying a plurality of elements to be sanitised, in turn, in batchwise manner to a sanitisation tunnel constituted by a series of in-line interconnected chambers and comprising a sanitisation chamber which is flanked by a first air lock chamber to which said elements are initially introduced and by a second air lock chamber so that each element spends a predetermined residence time in each of the first air lock chamber, the sanitisation chamber and the air lock chamber;
supplying vapour phase sanitisation medium to the sanitisation chamber while at least one said element is resident therein, with supply of said sanitisation medium to the sanitisation chamber being followed by bleeding thereof from the sanitisation chamber and being associated with leakage thereof into the first and second air locks as communication therebetween and the sanitisation chamber is effected, the supply, bleeding and leakage of sanitisation medium being controlled to be such as to maintain a relationship between concentration of sanitisation medium in the sanitisation chamber and time which possesses a square waveform;
rinsing with air the interiors of each of the air lock chambers after communication thereof with the sanitisation chamber; and
withdrawing said air from the air lock chambers and subjecting the air to the action of catalytic decomposition means to effect catalytic decomposition of vapour phase sanitisation medium entrained therein.

The preferred sanitisation medium is vapour phase hydrogen peroxide. The overall system in such case will have associated heating units and humidification units to ensure that operation within the system is kept within predetermined limits, preferably 20 to 60°C, more preferably 35 to 45°C, most preferably about 38°C, and a typical relative humidity in the range of 10 to 65 % when employing hydrogen peroxide as vapour phase sanitisation medium at about 30 % in the vapour phase supplied to the sanitisation chamber. By operating under such conditions, it is, in fact, possible to achieve sterilisation of elements passing through the sanitisation tunnel. Moreover, by operating under such conditions and avoiding use of mild steel and fabricating the equipment from stainless steel, accelerated oxidation of the stainless steel which will otherwise occur in the presence of vapour phase hydrogen peroxide is avoided.

For a better understanding of the invention and to show how the same can be carried into effect, reference will now be made by way of example only to the accompanying drawings wherein:
Figure 1 shows schematically plant for carrying out the respective stages of a sanitisation method embodying the invention,
Figure 2 shows a typical relationship between the amount of vapour phase sanitisation medium present in the sanitisation chamber and time, and
Figure 3 shows the preferred variation of vapour phase sanitisation medium concentration over the length of the sanitisation chamber.

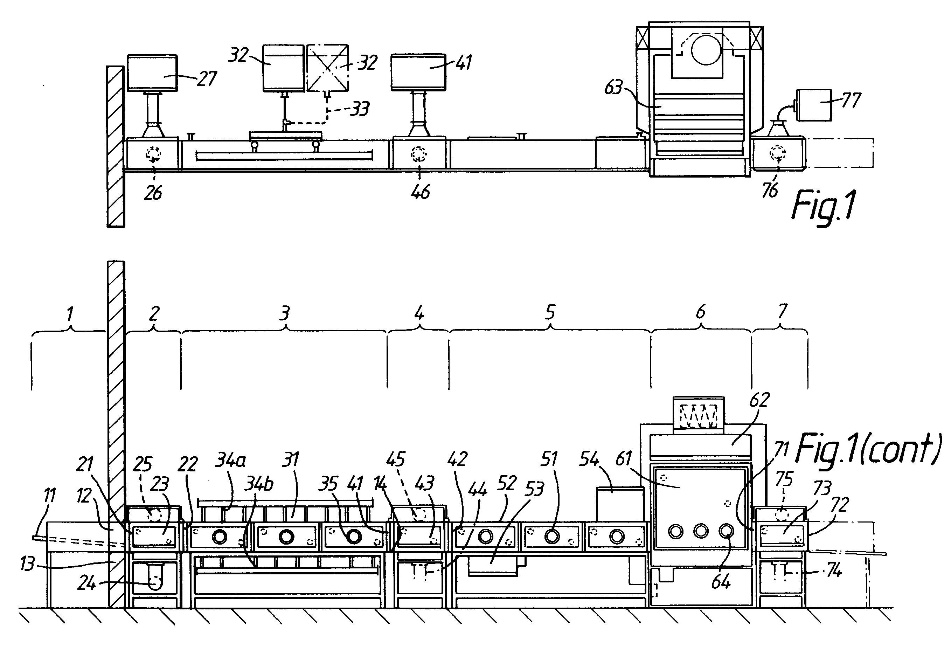

Referring first to Figure 1 of the drawings, there is shown a seven stage sanitisation arrangement of which the first stage shown is a line feed area 1 in which packs to be sanitised undergo preliminary unpacking, from outer wrappings, followed by covering with aluminised tape as a gas barrier before the packs are placed on a ramp 11 down which they are conveyed onto a conveyor 14 which they reach through via a door 12 adjacent a partition 13 and on which they are conveyed through the main sanitisation area. The sanitisation area is shown divided into six sections respectively numbered 2 to 7 each with its own conveyor and associated actuator for moving onto the respective conveyor packs to travel thereover. These features are not illustrated in the drawings.

The first section within the sanitisation area is a pass-in air lock 2 having inlet and outlet doors 21 and 22 respectively with an air lock chamber 23 therebetween. Inlet and outlet ducts 24 and 25 for rinsing air are indicated, with there being communication between the outlet duct 25 and an admission opening 26 to an arrangement 27 for the catalytic degradation of sanitisation medium which has been swept out of chamber 23 by rinsing air.

Once a package has been conveyed out of chamber 23 through door 22, it finds itself at the beginning of a sanitisation section 3 in which is disposed a square-sectioned sanitisation unit 31 of double walled construction which is supplied with vapour phase sanitisation medium from storage means 32 through a system of ducts 33 equipped with appropriate valves for switching flow or controlling flow as required. A plurality of openings 34a and 34b on the interior of the chamber allows vapour phase sanitisation medium to be admitted to the interior of the sanitisation tunnel to act on elements being conveyed therethrough and to be bled out of the chamber. Ports 35 are located in the wall of the sanitisation chamber at intervals corresponding to stationary positions taken up by elements in the tunnel as they travel therethrough in batch-wise manner and are equipped with gloves so that the position of packages may be adjusted, if necessary, by hand. Three ports are shown for three rest positions of such elements and the relative sizes of chamber 31 and air lock chamber 23 signifies a residence time in the former three times as long as the latter. In practice, the unit 31 and supply pipes thereto are heated to about 50°C to minimise condensation problems.

The next section is a pass-out air lock section 4 which is of essentially similar construction to air lock section 2 and in which component parts whose reference numerals commence with "2" in section 2 are here proceeded by the number "4".

Following the pass-out air lock system 4 is an accumulation section 5 shown with three ports 51 and able to accommodate the same number of packages as the sanitisation chamber 31. The accumulation section is formed as a tunnel 52 and is equipped with air entry and removal means 53 and 54 at the input and extract ends respectively which like most or all air ducts associated with the sanitisation include high energy particulate air filters, the latter having a catalytic decomposer for any residual sterilisation medium.

Following the accumulation section 5 is an operations isolator section 6 in which specific operations are to be carried out on the contents of the packages in a sterile environment. The operations isolator comprises a chamber 61 having an associated filling machine 62 fed by supply arrangement 63. Access by an operator to the interior of the filling machine is through ports 64 equipped with gloves for peeling back Tyvek panels. A path through the isolator chamber represents a continuation of the sterile tunnel in which the packages travel.

The final section is an exit air lock section 7 which is constructed like sections 2 and 4 with like parts receiving the same reference numerals as in section 2 but with the first number "2" being replaced by "7". From section 7, the packages travel out of the tunnel to be provided with replacement covers and to be labelled. Equipment for these stages is not shown.

The processes taking place in the various stages will now be described in greater detail as follows with specific reference to the handling of packages covered with Tyvek panels and containing syringe barrels to be filled and closed:-

### Line feed area 1

The line feed area is essentially an unpacking and debagging area in which cartons containing packs with Tyvek panels and disposed in outer packaging and/or shrink wrapped are emptied and stockpiled ready for feeding.

### Pass-in air lock 2

The packs are fed on ramp 11 to air lock chamber 23, the primary function of which is to maintain an air lock between the unpackaging area and the sterilisation chamber 31 to maintain over an eight hour period a requirement of 1 ppm maximum concentration of H₂O₂ in air to which an operator is exposed. Atmosphere and temperature conditions in the air lock chamber are conditioned so as not to interfere with the automatic requirements of the sterilisation system when there is communication between the two. In the air lock chamber, the package is exposed to a warm air stream, remaining static for about 30 seconds as a shower of warm air is passed over it. The warm air shower has the additional functions of assisting in conditioning of air temperature and relative humidity in the sterilisation tunnel and subjecting the surface temperature of the pack to a preliminary temperature increase. After a pack has left the pass-in air lock 2 through the outlet door 22 and the outlet door has been closed, an initial 15 second period is allowed for replacing the chamber air before supply of a further pack into the pass-in air lock through the inlet door 21. This cycle is repeated for each batch of packs en route to the sterilisation chamber itself. The outlet duct 25 from the pass-in air lock 2 includes a catalytic convertor 27 containing, for example palladium as active material to effect decomposition of hydrogen peroxide in the withdrawn air. For different sanitisation media, there may be different active catalysts for achieving degradation thereof.

### Sanitisation Section 3

The sole function of this stage is to surface disinfect the packs undergoing travel through the system on conveyor 14 to achieve 10³ kill, with 10⁶ kill being the preferred objective. Within the sanitisation chamber 31, the packages therein are exposed to vapour phase hydrogen peroxide for a predetermined time. The supply from storage means 32 for the hydrogen peroxide typically used is such as to compensate for relative humidity influences and temperature influences as a result of temperature controlled air leaking into the sanitisation chamber 31 when the doors 22 and 41 which flank it are open.

### Pass-out air lock 4

The function of this stage is to separate the sanitisation chamber 31 itself from the accumulation section which is to follow directly on. An air shower is again used to reduce vapour phase hydrogen peroxide residuals to a minimum prior to ejection of a pack to the accumulation section and receipt of a subsequent pack. Temperature and relative humidity control is again provided so as not to affect atmospheric requirements of the sanitisation chamber. It is preferred here to employ a residence period in the warm air shower which is longer than that in the air lock 2 owing to the greater amount of residual vapour phase hydrogen peroxide carried through. Typically, a residence period of 45 seconds would be utilised here. A catalytic convertor is also provided to decompose hydrogen peroxide from the air entering the air lock section 4 from the sanitisation chamber 31.

Operation of the air lock doors, residence time within sections 2, 3 and 4 and supply of sanitisation medium are controlled by sensors indicating the admission of packs to particular sections and operated on a time related basis to provide for variation in concentration of vapour pressure hydrogen peroxide in the sanitisation section over the period of residence of a pack therein to possess a square wave form relationship while at all times keeping the pressures within respective chambers negative with respect to the stages before and after.

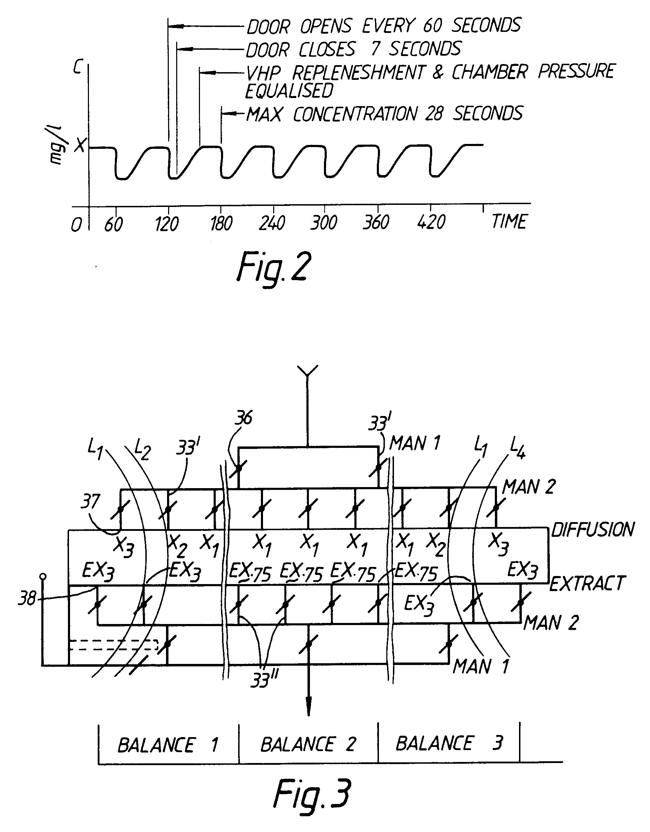

Thus, Figure 2 shows a series of square wave forms recurring every 60 seconds. As the door 22 from air lock chamber 23 is opened, there is a pressure leak from the sanitisation chamber 31 into the air lock chamber 23. A pack is accelerated into the sanitisation chamber. When door 22 is verified clear, it closes and the pack now in sanitisation chamber 31 is exposed to H₂O₂ for a predetermined time while, at the same time, gas supply through ducts 33 is such as to compensate for relative humidity changes and effect of entry of temperature controlled air leakage into chamber 31 when the door 22 was open. Each H₂O₂ concentration cycle follows the wave form indicated and how work-up vapour phase H₂O₂ maintains the neutralising level required at any one position in the sterilisation chamber.

In fact, Figure 3 indicates how H₂O₂ concentration varies within the sterilisation chamber. A series of supply ducts 33' and extraction ducts 33'' and manifolds 36 associated with a sanitisation chamber 31 is shown, including diffusion inlets 37 and extraction outlets 38. Two notional concentration fronts L₁, L₂ are shown at the entry side to the chamber 31 and two notional fronts L₃, L₄ are shown the exit side of the chamber 31. There is alternation of phase between L₁, L₂, L₃, L₄ every 30 seconds in respect of H₂O₂ concentration.

In this way concentrations are maintained overall during door open and door closed phases of operation. Because the sanitisation section is maintained at at least 2 pressures below that of air-locks 2 and 4, this will enable there to be acceptance of over pressure leaks from air locks when the respective doors 22 and 41 are open.

Extraction of H₂O₂ i.e. bleed H₂O₂ from sterilisation chamber 31 needs to take place near the doors 22 and 41 to a greater extent when they are open. This is to help reduce over pressure and to reduce make-up vapour phase H₂O₂ supply requirements. In turn, the concentration of H₂O₂ supplied to the door areas is to be higher than that delivered elsewhere to the chamber 31.

### Accumulator 5

The primary function of this section is to provide a dwell space to dissipate any residual vapour phase hydrogen peroxide and to provide a reservoir of packs for the personnel operating the subsequent steps. A single path system is preferably employed to avoid humidity build up from H₂O emitted from the breakdown of H₂O₂.

### Operations isolator 6

This is an open section, that is not of minimal cross-section tunnel form, into which enters a filling machine 62. In this open section, the packs are accessible to the operator through the ports to enable the operator to carry out the following sequence of operations:-
1. The Tyvek cover on incoming packs is peeled off to expose trays of syringe barrels are removed from the packs.
2. A previously filled tray is removed from the filling machine.
3. The newly exposed barrel tray is inserted into the filling machine and the barrels are filled.

Simultaneously with the travel of each pack through the sanitisation tunnel, there will have been travel of a cradle containing stoppers for the barrels. The transport cradle may be suspended within the overall tunnel system for travel purposes. The handling of the cradles follows a like routine to that for the packages and their contents and, in the operations isolator it is then possible to remove the stoppers from the cradles and insert them in the barrels. Each empty cradle is removed from the operations isolator at the same time as the filled tray in its original pack which is ready for repackaging for onward transport - provided in the operations isolator chamber are inlet nozzles for further supply of sterilising vapour pressure hydrogen peroxide.

### Exit air lock 7

This air lock simply provides an air lock chamber 73 between the operations isolator area and the atmosphere to which the filled packs are removed. Any residual hydrogen peroxide which has entered the air lock chamber will be removed by means of rinsing air in the same manner as hydrogen peroxide in sections 2 and 4.

## Claims

1. A continuous sanitisation method which comprises supplying a plurality of elements to be sanitised, in turn, in batchwise manner to a sanitisation tunnel constituted by a series of in-line interconnected chambers (2-7) and comprising a sanitisation chamber (3) which is flanked by a first air lock chamber (2) with inlet and outlet doors (21, 22) to which said elements are initially introduced and by a second air lock chamber (4) with inlet and outlet doors (41, 42) so that each element spends a predetermined residence time in each of the first air lock chamber, the sanitisation chamber and the second air lock chamber;
supplying vapour phase sanitisation medium to the sanitisation chamber while at least one said element is resident therein, with supply of said sanitisation medium to the sanitisation chamber being followed by bleeding thereof from the sanitisation chamber and being associated with leakage thereof into the first and second air locks as communication therebetween and the sanitisation chamber is effected, the supply, bleeding and leakage of sanitisation medium being controlled to be such as to maintain a relationship between concentration of sanitisation medium in the sanitisation chamber and time which possesses a square waveform;
rinsing with air the interiors of each of the air lock chambers after communication thereof with the sanitisation chamber; and
withdrawing said air from the air lock chambers and subjecting the air to the action of catalytic decomposition means to effect catalytic decomposition of vapour phase sanitisation medium entrained therein.

2. A method as claimed in claim 1, wherein sanitisation is carried out utilising vapour phase hydrogen peroxide.

3. A method as claimed in claim 2, wherein the temperature within the sanitisation tunnel is maintained at from 20 to 60°C, preferably from 35 to 45°C.

4. A method as claimed in claims 2 or 3, wherein a relative humidity is maintained within the tunnel which is in the range from 10 to 65%, the sanitisation medium containing 30% concentration of hydrogen peroxide.

5. A method as claimed in any one of claims 2 to 4, wherein air from the air lock chambers is passed over palladium to effect decomposition of hydrogen peroxide therein.

6. A method as claimed in any one of claims 1 to 5, wherein a plurality of notional sanitisation medium concentration fronts is provided at each of the entry side and the exit side of the sanitisation chamber with alternation of phase taking place in respect of each front at predetermined intervals.

7. A method as claimed in any preceding claim wherein, while still within the sanitisation tunnel, the elements are worked upon in at least one work station chamber itself followed by a further said air lock chamber.

8. A method as claimed in claim 7, wherein said elements are empty containers and filling means therefor is operated in a said work station chamber.

9. A method as claimed in claim 7 or 8, wherein manual working of the elements takes place at a said work station chamber by means of a glove arrangement accessible from outside the work station chamber.

10. A method as claimed in claim 8 or 9, wherein the alignment of the elements takes place manually in an accumulator section preceding the work station chamber by means of a glove arrangement accessible from outside the accumulator section.

## Patentansprüche

1. Kontinuierliches Verfahren zur Sterilisation (sanitisation), das die stapelartige Zufuhr einer Vielzahl zu sterilisierender Elemente nacheinander zu einem Steriltunnel umfaßt, der aus einer Folge von sich in Reihe befindlichen miteinander verbundenen Kammern (2-7) besteht, und der eine Sterilisationskammer (3), flankiert von einer ersten Luftschleusenkammer (2) mit Einlaß- und Auslaßtüren (21, 22), in die diese Elemente am Anfang eingeleitet werden, und von einer zweiten Luftschleusenkammer (4) mit Einlaß- und Auslaßtüren (41, 42), umfaßt, so daß jedes Element eine vorherbestimmte Verweilzeit jeweils in der ersten Luftschleusenkammer, der Sterilisationskammer und der zweiten Luftschleusenkammer zubringt;
wobei der Sterilisationskammer Sterilisationsmedium in Dampfphase zugeführt wird, während in ihr mindestens eines dieser Elemente ruht, wobei die Zufuhr dieses Sterilisationsmediums zur Sterilisationskammer von einer Ableitung desselben aus der Sterilisationskammer gefolgt ist und assoziiert wird mit Entweichen desselben in die erste und zweite Luftschleuse bei Verbindung zwischen diesen und der Sterilisationskammer, wobei Zufuhr, Ableitung und Entweichen des Sterilisationsmediums so gesteuert werden, daß zwischen der Konzentration des Sterilisationsmediums in der Sterilisationskammer und der Zeit eine Relation beibehalten wird, die durch eine Rechteckkurvenform beschrieben wird;
wobei das Innere jeder der Luftschleusenkammern nach Verbindung derselben mit der Sterilisationskammer mit Luft gereinigt wird; und
wobei diese Luft den Luftschleusenkammern entzogen und die Luft der Wirkung von katalytischem Zersetzungsmittel ausgesetzt wird, um katalytische Zersetzung des darin mitgeführten Sterilisationsmediums in Dampfphase zu bewirken.

2. Verfahren nach Anspruch 1, worin die Sterilisation unter Verwendung von Wasserstoffperoxyd in Dampfphase durchgeführt wird.

3. Verfahren nach Anspruch 2, worin die Temperatur innerhalb des Steriltunnels bei zwischen 20 und 60°C, vorzugsweise zwischen 35 und 45°C, beibehalten wird.

4. Verfahren nach einem der Ansprüche 2 oder 3, worin innerhalb des Tunnels ein relativer Feuchtigkeitsgehalt in der Größenordnung von zwischen 10 und 65% beibehalten wird, wobei das Sterilisationsmedium eine 30%ige Konzentration an Wasserstoffperoxyd enthält.

5. Verfahren nach einem der Ansprüche 2 bis 4, worin Luft aus den Luftschleusenkammern über Palladium geleitet wird, um die Zersetzung des Wasserstoffperoxyds darin zu bewirken.

6. Verfahren nach einem der Ansprüche 1 bis 5, worin sich jeweils auf der Einlaßseite und Auslaßseite der Sterilisationskammer eine Vielzahl von angenommenen Konzentrationsfronten des Sterilisationsmediums befindet, wobei in vorherbestimmten Intervallen ein Phasenwechsel hinsichtlich jeder Front stattfindet.

7. Verfahren nach einem der vorgenannten Ansprüche, worin die Elemente, während sie sich noch im Steriltunnel befinden, in zumindest einer Arbeitsplatzkammer, die wiederum von einer weiteren besagten Luftschleusenkammer gefolgt wird, bearbeitet werden.

8. Verfahren nach Anspruch 7, worin diese Elemente leere Behälter sind und Füllung dafür in einer besagten Arbeitsplatzkammer durchgeführt wird.

9. Verfahren nach Anspruch 7 oder 8, worin manuelle Bearbeitung der Elemente in einer besagten Arbeitsplatzkammer mittels einer von außerhalb der Arbeitsplatzkammer erreichbaren Handschuhvorrichtung erfolgt.

10. Verfahren nach Anspruch 8 oder 9, worin die Ausrichtung der Elemente manuell in einer Sammelsektion vor der Arbeitsplatzkammer mittels einer von außerhalb der Sammelsektion erreichbaren Handschuhvorrichtung erfolgt.

## Revendications

1. Procédé de stérilisation en continu qui consiste:
à fournir une pluralité d'éléments à stériliser, tour à tour, lot par lot, à un tunnel de stérilisation constitué par une série de chambres (2-7) raccordées les unes aux autres, en ligne, et comprenant une chambre de stérilisation (3) qui est flanquée par une première chambre formant sas (2) munie de portes d'entrée et de sortie (21, 22) et dans laquelle lesdits éléments sont initialement introduits, et par une deuxième chambre formant sas (4) munie de portes d'entrée et de sortie (41,42) de telle sorte que chaque éléments séjourne un temps prédéterminé dans chacune des chambres, à savoir la première chambre formant sas, la chambre de stérilisation et la deuxième chambre formant sas;
à fournir un milieu de stérilisation en phase vapeur à la chambre de stérilisation pendant qu'au moins un desdits éléments y séjourne, la fourniture dudit milieu de stérilisation à la chambre de stérilisation étant suivie par une purge de ce milieu de la chambre de stérilisation et étant associée à une fuite de ce milieu dans les premier et deuxième sas lorsqu'une communication entre ces sas et la chambre de stérilisation est établie, la fourniture, la purge et la fuite du milieu de stérilisation étant commandées de manière à maintenir, entre la concentration du milieu de stérilisation se trouvant dans la chambre de stérilisation et le temps, une relation qui est représentée par une forme d'onde carrée;
à rincer avec de l'air l'intérieur de chacune des chambres formant sas après sa mise en communication avec la chambre de stérilisation; et
à évacuer ledit air des chambres formant sas et à soumettre l'air à l'action d'un moyen de décomposition catalytique pour effectuer une décomposition catalytique du milieu de stérilisation en phase vapeur qui y est entraîné.

2. Procédé selon la revendication 1, dans lequel on effectue la stérilisation en utilisant du peroxyde d'hydrogène en phase vapeur.

3. Procédé selon la revendication 2, dans lequel on maintient la température à l'intérieur du tunnel de stérilisation dans un intervalle allant de 20 à 60°C, de préférence de 35 à 45°C.

4. Procédé selon les revendications 2 ou 3, dans lequel on maintient à l'intérieur du tunnel une humidité relative qui est comprise dans un intervalle allant de 10 à 65%, le milieu de stérilisation contenant du peroxyde d'hydrogène en une concentration de 30%.

5. Procédé selon l'une quelconque des revendications 2 à 4, dans lequel on fait passer l'air provenant des chambres formant sas sur du palladium pour effectuer la décomposition du peroxyde d'hydrogène qui s'y trouve.

6. Procédé selon l'une quelconque des revendications 1 à 5, dans lequel une pluralité de fronts de concentration théorique de milieu de stérilisation est fournie à chacun des côtés d'entrée et de sortie de la chambre de stérilisation, une alternance de phase ayant lieu en ce qui concerne chaque front à des intervalles prédéterminés.

7. Procédé selon l'une quelconque des revendications précédentes, dans lequel, pendant qu'ils se trouvent encore dans le tunnel de stérilisation, on agit sur les éléments, dans au moins une chambre formant poste de travail, elle-même suivie par une autre chambre formant sas.

8. Procédé selon la revendication 7, dans lequel lesdits éléments sont des récipients vides et on actionne un moyen de remplissage, destiné à ces récipients, dans ladite chambre formant poste de travail.

9. Procédé selon la revendication 7 ou 8, dans lequel on agit manuellement sur les éléments dans ladite chambre formant poste de travail au moyen d'un système de gants, accessible de l'extérieur de la chambre formant poste de travail.

10. Procédé selon la revendication 8 ou 9, dans lequel l'alignement des éléments a lieu manuellement dans une section d'accumulation précédant la chambre formant poste de travail au moyen d'un système de gants, accessible de l'extérieur de la section d'accumulation.
